(19) Europäisches Patentamt
European Patent Office
Office européen des brevets

(11) Publication number: 0 327 364 A1

(12) # EUROPEAN PATENT APPLICATION

(21) Application number: 89301011.6

(22) Date of filing: 02.02.89

(51) Int. Cl.[4]: C 07 D 471/04
A 61 K 31/435
//(C07D471/04,221:00,205:00)

(30) Priority: 05.02.88 US 152777

(43) Date of publication of application:
09.08.89 Bulletin 89/32

(84) Designated Contracting States:
AT BE CH DE ES FR GB GR IT LI LU NL SE

(71) Applicant: ELI LILLY AND COMPANY
Lilly Corporate Center
Indianapolis Indiana 46285 (US)

(72) Inventor: Daugherty, Byron Wade
8319 S. East Street
Indianapolis Indiana 46227 (US)

(74) Representative: Hudson, Christopher Mark et al
Erl Wood Manor
Windlesham Surrey GU20 6PH (GB)

Claims for the following Contracting States: ES + GR

(54) **Crystalline beta-lactam solvate.**

(57) The crystalline 2.5 hydrate of 7β-[2′-(R)-2′-(m-(methylsulfonamido)phenyl)-2′-aminoacetamido]-3-chloro-3-(1-carbadethiacephem)-4-carboxylic acid is an antibiotic with superior pharmaceutical elegance. Pharmaceutical formulations of the crystalline 2.5 hydrate are also described.



Bundesdruckerei Berlin

**Description**

## CRYSTALLINE β-LACTAM SOLVATE

This invention relates to a novel solvate of a β-lactam antibiotic, more particularly to a novel crystalline solvate of a 1-carbacephalosporin, having an unusual degree of hydration.

The β-lactam antibiotic of the Formula (I)

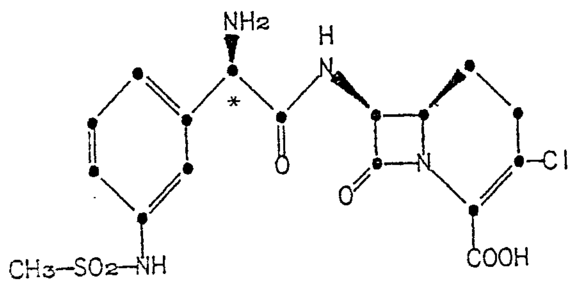

(I)

where the asterisk indicates R-absolute stereochemistry, is a potent new orally-active antibiotic. The antibiotic is generically described in U.S. Patent No. 4,335,211 and European Patent No. 14,476. In addition, the compound is specifically exemplified and claimed in European Patent Application No. 266896. For brevity's sake, the anhydrous form of the compound of the above formula will be referred to by the Lilly serial number "LY228238".

The instant invention is directed to the solvate of LY228238 having 2.5 molecules of water associated with each molecule of LY228238. For brevity, this polyhydrate will be referred to as LY228238 2.5 hydrate ("2.5 hydrate"). The compound is a pharmaceutically elegant 2.5 hydrate of the parent compound. The crystalline 2.5 hydrate is stable over a wide relative humidity range, (i.e., approximately 30% to approximately 100%) and especially in the humidity range encountered in production and subsequent storage (approximately 30%) of the finished (commercial) form containing the compound. Furthermore, the crystalline 2.5 hydrate has a superior bulk density to the amorphous form of the parent compound, and thus is a superior form for filling capsules.

The LY228238 crystalline 2.5 hydrate has the following X-ray powder diffraction pattern:

Table

| d | $I/I_1$ |
|---|---|
| 23.86 | .06 |
| 14.74 | .12 |
| 10.23 | .07 |
| 9.46 | 1.00 |
| 6.89 | .09 |
| 6.42 | .15 |
| 6.30 | .15 |
| 5.89 | .21 |
| 5.22 | .26 |
| 4.88 | .05 |
| 4.61 | .12 |
| 4.58 | .16 |
| 4.40 | .80 |
| 4.29 | .84 |
| 4.09 | .36 |
| 4.01 | .42 |
| 3.12 | .10 |

The above pattern was obtained with nickel-filtered copper radiation (Cu:Ni) of wavelength $\lambda = 1.54056$ Å. The interplanar spacings are in the column denoted as "d" and the relative intensities are in the column "$I/I_1$". The 2.5 hydrate is a white microcrystalline solid. The compound of Formula (I) can be in the form of a zwitterion, a form which is also encompassed by the instant invention. For brevity's sake, the compound of Formula (I) will be referred to as "LY228238", and the compound of the instant invention as either "LY228238 2.5 hydrate", the "crystalline 2.5 hydrate" or, more simply, the "2.5 hydrate".

The "2.5 hydrate" of this invention, in fact, appears to be a compound or complex wherein the molar ratio of water of hydration to LY228238 is 2.5:1. This ratio appears to be constant throughout normally encountered humidity ranges. For the purpose of this disclosure, the compound as characterized by the X-ray powder diffraction pattern of the Table shall be referred to simply as a 2.5 hydrate.

The 2.5 hydrate can be made from the anhydrate or various alcohol or other solvates of the parent compound. The preferred starting material is either the anhydrate or the ethanol solvate of LY228238.

As such, this invention provides a process for preparing the crystalline 2.5 hydrate of the compound of Formula (I) which comprises freeze-drying an aqueous solution containing a compound of Formula (I) and crystallizing the resulting solid from water.

The above starting materials are first dissolved in water to which a minimum amount of acid, generally 6N (or more dilute) hydrochloric acid or preferably acetic acid has been added. The preferred method of preparing the 2.5 hydrate of this invention employs a 1% acetic acid in water solution. After solution is effected, the solution is filtered and freeze-dried. The resulting powder is then crystallized from water. Approximately 5-20 grams of water, preferably about 10 grams, is employed for every gram of solid. Crystallization is best accomplished by effecting solution at 30-50° C, and then allowing the solution to sit at room temperature (20-30° C) until crystal formation is completed. The resulting product can then be collected by conventional means, such as vacuum filtration or centrifugation. The crystals may be washed with a small volume of water if desired and dried, for example, in a cleaned air oven.

As stated above, a further aspect of this invention is a pharmaceutical formulation comprising as an active ingredient a crystalline 2.5 hydrate of the compound of Formula (I) associated with one or more pharmaceutically-acceptable carriers, vehicles or excipients therefor. These pharmaceutical compositions are useful for the control of gram-positive and gram-negative bacterial infections in warm-blooded animals.

With regard to compositions for oral administration (such as tablets and capsules), the term "suitable carrier, vehicle or excipient" means common excipients such as binding agents, for example, syrup, acacia, gelatin, sorbitol, tragacanth, polyvinylpyrrolidine (Povidone), methylcellulose, ethylcellulose, sodium carboxymethylcellulose, hydroxypropylmethylcellulose, sucrose, and starch; fillers and carriers, for example corn starch, gelatin, lactose, sucrose, microcrystalline cellulose, kaolin, mannitol, dicalcium phosphate, sodium chloride, and alginic acid; disintegrators such as croscarmellose sodium, microcrystalline cellulose, corn starch, sodium starch glycolate, alginic acid and mutable wetting agents such as sodium lauryl sulfate; and lubricants such as magnesium stearate and other metallic stearates, stearic acid, silicone fluid, talc, waxes, oils, and colloidal silica. Flavoring agents such as peppermint, oil of wintergreen, cherry flavoring, or the like can also be used. It may be desirable to add a coloring agent to make the dosage form more aesthetically pleasing in appearance or to help identify the product. The tablets may also be coated by methods well known in the art.

The pharmaceutical compositions of the present invention may also be in the form of oral liquid preparations, which may be either a) aqueous or oily suspensions, solutions, emulsions or syrups; or b) a dry powder to be reconstituted with water or another suitable vehicle before use. When used in conjunction with such oral liquid preparations, the term "suitable carrier, vehicle or excipient" means conventional additives such as suspending agents such as acacia, methyl cellulose, hydroxyethyl cellulose, carboxymethyl cellulose, cellulose with sodium carboxymethyl cellulose (Avicel®), xantham gum, or starch; sweeteners such as sucrose, syrup, glucose, saccharin, sorbital, or aspartame; wetting agents such as sodium lauryl sulfate, silicone oil, the various Pluromics® surfactants, or glycerin; preservatives such as methyl, propyl, or butyl p-hydroxy benzoates, or sorbic acid; dyes, flavors and salts such as sodium chloride, citric acid, oil of wintergreen or sodium citrate; and waters, oils, and esters such as almond oil, fractionated coconut oil, hydrogenated caster oil, lecithin, aluminum stearate, and the like.

Topical compositions can be formulated with "suitable carriers, vehicles or excipients" such as hydrophobic or hydrophilic bases. Such bases include ointments, creams or lotions.

Veterinary pharmaceutical compositions of the antibiotic compounds may be administered in the feed or the drinking water of farm animals. Alternatively, the compounds can be formulated as intramammary preparations with "suitable carriers, vehicles or excipients" such as long- or quick-release bases.

The crystalline 2.5 hydrate of LY228238 can also be formulated in unit dosage form in sterile vials, sterile plastic pouches containing a port with a septum, or sterile, hermetically sealed ampoules. The amount of the crystalline 2.5 hydrate compound per unit dosage may vary from about 100 milligrams to about 10 grams. A preferred amount is from 200 to 500 milligrams per unit dosage.

A "therapeutically effective amount" of the crystalline 2.5 hydrate compound of this invention is from approximately 2.5 mg to about 70 mg of compound per kilogram of body weight per dose. This amount generally totals from about 200 mg to about 7 grams per day for an adult human.

During a course of treatment with the instant crystalline 2.5 hydrate antibiotic, the antibiotic compound can be administered in a single daily dose or in multiple doses per day. The treatment regime may require administration over extended periods of time, for example, for several days or for from two to three weeks. The amount administered per dose or the total amount administered will depend on such factors as the nature and severity of the infection, the age and general health of the patient, and the tolerance to the crystalline 2.5 hydrate antibiotic compound of Formula I of both the patient and the microorganism or microorganisms involved in the infection.

The starting material (LY228238) for the compound of Formula I is made by acylating a 7β-amino ("nucleus")

compound of the Formula II

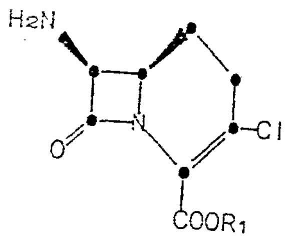

II

then removing the carboxy-protecting group represented above as $R_1$.

The carboxy-protecting group $R_1$ of formula II is a conventional carboxy-protecting group and preferably one which is not sterically hindered. Examples of such groups are benzyl and substituted benzyl groups such as 4-methoxybenzyl, 4-nitrobenzyl, 4-methylbenzyl, 3,5-dimethylbenzyl, and 4-chlorobenzyl; silyl group such as a trialkylsilyl group (trimethylsilyl); and halo-substituted alkyl groups such as the 2,2,2-trichloroethyl, 2,2,2-tribromoethyl, and 2-iodoethyl groups. A preferred ester group is the benzyl or a substituted benzyl ester group.

The methods for the acylation of the 7β-amino compounds of Formula II with an acyl side chain are similar to the methods for the acylation of 6-aminopenicillanic acid, 7-aminodesacetoxycephalosporanic acid, and 7-aminocephalosporanic acid. One method is to simply combine the 7β-amino nucleus with an acid chloride or acid bromide in the presence of an acid scavenger. The acid chloride or acid bromide may be formed in situ. Another method is to combine the 7β-amino nucleus with the free carboxylic acid form of the side chain (or its acid salt) and a condensing agent. Suitable condensing agents include N,N′-disubstituted carbodiimides such as N,N′-dicyclohexylcarbodiimide, N,N′-diethylcarbodiimide, N,N′-di(n-propyl)carbodiimide, N,N′-di(iso-propyl)carbodiimide, N,N′-diallylcarbodiimide, N,N′-bis(p-dimethylaminophenyl)carbodiimide, N-ethyl-N′-(4″-ethylmorpholinyl)carbodiimide, and the like. Other suitable carbodiimide condensing agents are disclosed by Sheehan in U.S. Patent No. 2,938,892 and by Hofmann et al. in U.S. Patent No. 3,065,224. Azolides, such as N,N′-carbonyldiimidazole and N,N′-thionyldiimidazol, may also be used as condensing agents. Dehydrating agents such as phosphorus oxy chloride, the alkoxyacetylenes, and 2-halogenopyridinium salts (such as 2-chloropyridinium methyl iodide, 2-fluoropyridinium methyl iodide, and the like) may be used to couple the free acid or its acid salt with the 7β-amino nucleus.

Another acylation method entails first converting the free carboxylic acid form (or the corresponding salt) of the acyl side chain to the corresponding active ester derivative, which is in turn used to acylate the nucleus. The active ester derivative is formed by esterifying the free acid form with groups such as p-nitrophenol, 2,4-dinitrophenol, trichlorophenol, pentachlorophenol, 2-chloro-4,6-dimethoxytriazene, N-chlorosuccinimide, N-chloro maleic imide, N-chlorophthalimide, 1-hydroxy-1H-benzotriazole or 1-hydroxy-6-chloro-1H-benzotriazole. The active ester derivatives can also be mixed anhydrides, which are formed with groups such as methoxycarbonyl, ethoxycarbonyl, iso-butoxycarbonyl, trichloromethylcarbonyl, and iso-but-2-ylcarbonyl, and the carboxylic acid of the acyl side chain. The mixed anhydrides are synthesized by acylating the carboxylic acid of the acyl side chain.

Alternatively, the 7β-amino nucleus can be acylated with the N-ethoxycarbonyl-2-ethoxy-1,2-dihydroquinoline (EEDQ) derivative of the acyl side chain. In general, the free acid form of the acyl side chain and EEDQ are reacted in an inert, polar organic solvent (such as tetrahydrofuran, acetonitrile, and the like). The resultant EEDQ derivative is used in situ to acylate the 7β-amino nucleus.

Yet another method of acylating the 7β-amino compounds entails the use of an enzymatically-assisted process. Such a process is described in Hashimoto et al., U.S. Patent No. 4,335,211, issued June 15, 1982, herein incorporated by reference.

A preferred method of acylation in general is to first silylate the nucleus with, for example, N,N′-bis(trimethylsilyl)urea (BSU) in DMF. The DMF solution is cooled to a low temperature (-45° C to -50° C) then pyridine and the hydrochloride salt of the acid chloride derivative of an amino-protected m-methylsulfonamido-phenylglycine are added. The acylation is quenched by the addition of strong (5 or 6N) hydrochloric acid, and filtered. The acylated product is then recovered by adjusting the pH of the reaction solution to approxiately 6 (more preferably 6.1) by the addition of a base such as triethylamine. The resulting crystals are collected by filtration.

The amino- and carboxy-protecting groups are removed by methods well known in the art. Examples of conditions for the removal of these two types of protecting groups can be found in standard works on the subject, such as E. Haslam, "Protective Groups in Organic Chemistry", J.G.W. McOmie, Ed., Plenum Press, New York, N.Y., 1973, Chapters 2 and 5, and T.W. Greene, "Protective Groups in Organic Synthesis", John Wiley and Sons, New York, N.Y., 1981, Chapters 5 and 7, respectively.

Examples of procedures for the removal of amino- and carboxy-protecting groups can also be found in the Experimental Section. For example, the preferred t-butoxycarbonyl amino-protecting group was removed with

trifluoroacetic acid, and the p-nitrobenzyl carboxy-protecting group was removed by hydrogenolysis.

The 7β-amino 3-chloro 1-carba-1-dethiacephem compound of Formula II are synthesized from the corresponding 3-hydroxy compounds in accord with the process diagrammed below in Scheme 1:

Scheme 1

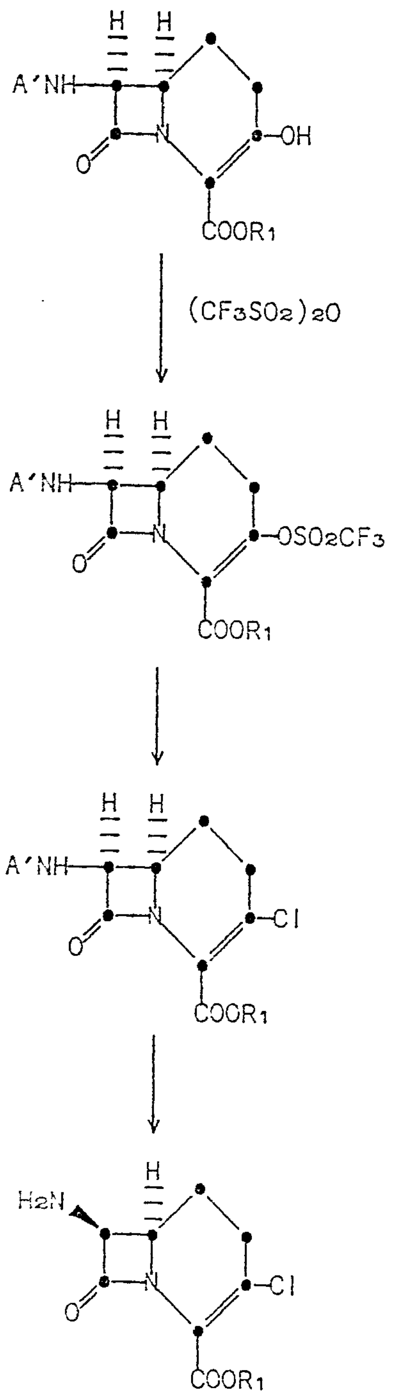


III

IIIa

IIA

II

In the above Scheme 1, $R_1$ is a carboxy-protecting (as described above for Formula II) and A is either an amino-protecting group or an acyl group of the formula
R-CO-
wherein R is:
hydrogen, $C_1$ to $C_6$ alkyl, $C_1$ to $C_6$ alkyl substituted by cyano, carboxy, halogen, amino, $C_1$ to $C_4$ alkoxy, $C_1$ to $C_4$ alkylthio, or trifluoromethylthio;
a phenyl or substituted phenyl group represented by the formula

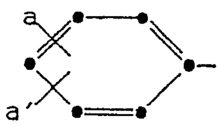

wherein a and a′ independently are hydrogen, halogen, protected hydroxy, $C_1$ to $C_4$ alkoxy, $C_1$ to $C_4$ alkanoyloxy, $C_1$ to $C_4$ alkyl, $C_1$ to $C_4$ alkylthio, protected amino, $C_1$ to $C_4$ alkanoylamino, $C_1$ to $C_4$ alkylsulfonylamino, protected carboxy, carbamoyl, protected hydroxymethyl, protected aminomethyl, or protected carboxymethyl;
a group represented by the formula

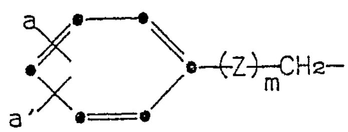

wherein a and a′ have the same meanings as defined above, Z is O or S, and m is 0 or 1;
a heteroarylmethyl group represented by the formula
$R_3$-$CH_2$-
wherein $R_3$ is thienyl, furyl, benzothienyl, benzofuryl, indolyl, triazolyl, tetrazolyl, oxazolyl, thiazolyl, oxadiazolyl, thiadiazolyl, and such heteroaryl groups substituted by protected amino, protected hydroxy, halogen, $C_1$ to $C_4$ alkyl, $C_1$ to $C_4$ alkoxy, or $C_1$ to $C_4$ alkylsulfonylamino groups;
a substituted methyl group represented by the formula
$R_4\text{-}\underset{\substack{|\\ Q}}{CH}\text{-}$
wherein $R_4$ is cyclohex-1,4-dienyl, a phenyl group or a substituted phenyl group represented by the formula

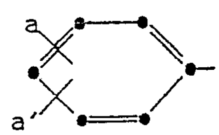

wherein a and a′ have the above defined meanings, or $R_4$ is $R_3$ as defined above, and Q is protected hydroxy, $C_1$ to $C_4$ alkanoyloxy, protected carboxy, sulfo, or protected amino.

The sulfonylation of the 3-hydroxy group represented by the first reaction in above Scheme I (Formula III→Formula IIIa) is carried out in an inert solvent at a temperature between about 0°C and about 35°C in the presence of a tertiary amine. Amines which are suitable include triethylamine, tri-(n-butyl)amine, pyridine, t-butyldiethylamine, di(isopropyl)ethylamine, and like amines. Hindered trialkylamines are preferred. The acylating reagent can be trifluoromethanesulfonic anhydride (triflic anhydride), trifluoromethanesulfonyl chloride (triflic chloride) or other suitable acid derivatives of trifluoromethanesulfonic acid. Inert solvents useful in the process are the halogenated hydrocarbons such as chloroform, methylene chloride, trichloroethane, and the like; ether solvents such as tetrahydrofuran; esters such as ethyl acetate; or other inert solvents such as acetonitrile.

The triflic esters (Formula IIIa) are recovered from the reaction mixture by conventional isolation methods, such as by extraction.

During the acylation any reactive groups in the side chain group R also capable of acylation is desirably protected. For example, any amino group substituents are protected with a conventional amino-protecting group to prevent amide formation in competition with the desired sulfonylation formation.

The second (chlorination) reaction in the above Scheme 1 (Formula IIIa→Formula IIa) proceeds with lithium chloride in an aprotic solvent at a temperature between about 60°C and about 95°C.

Aprotic polar solvents which can be used in the chlorination reaction are dimethylformamide, di methylacetamide, N-methylpyrrolidone, acetonitrile, and like solvents. Dimethylformamide is a preferred solvent.

Preferably the reaction is carried out at a temperature between about 75°C and about 85°C with an excess of the stoichiometric amount of the lithium chloride salt.

A preferred carboxy-protecting group at $R_2$ is the benzyl or a substituted benzyl group.

Following completion of the chlorination reaction the 3-chloro-1-carba-3-cephem ester is recovered from the reaction mixture by conventional isolation methods and is purified by chromatography.

As with the first reaction in Scheme 1, during the chlorination reaction it is desirable to protect any amino groups present in the starting material. In an example of the reaction, benzyl 7β-phenoxyacetylamino-3-trifluoromethylsulfonyloxy-1-carba-1-dethiacephem-4-carboxylate is dissolved in dimethylformamide and an excess (such as a 3-4 molar excess) of lithium chloride is added. The solution is stirred and heated to a temperature of about 80°C for about 5-6 hours. The progress of the chlorination can be followed by thin layer chromatography of a small aliquot periodically removed from the reaction mixture. When the reaction is completed the mixture is diluted with a water-immiscible organic solvent, washed with water, dried, and evaporated. The crude product (benzyl 7β-phenoxyacetylamino-3-chloro-1-carba-1-dethiacephem-4-carboxylate) is purified by chromatography (for example, over silica gel).

The final reaction in the above Scheme 1, which is either the removal of the amino-protecting group or the cleavage of the amido group represented by the partial formula "A′NH-" (compound IIa→Compound II) are reactions well known in the art. The removal of amino-protecting groups are taught in the references mentioned above for the acylation and subsequent deprotection of the compounds of Formula II.

The conversion of the 3-hydroxy compound (Formula III) to the 3-chloro compound (Formula IIA) as depicted above in Scheme 1 is also described in David A. Evans et al., U.S. Patent No. 4,673,737, issued June 16, 1987, herein incorporated by reference.

The methods for cleaving the amide bond of a 7-(amido) side chain are well known in the art. One such method employs nitrosyl chloride, as exemplified in M. Stamper et al., U.S. Patent No. 3,507,862, issued April 21, 1970, herein incorporated by reference. Another method uses phosphorus pentachloride in the presence of a (preferably nitrogen) base. The latter process is described in, for example, R. Chauvette, U.S. Patent No. 3,549,628, issued December 22, 1970, B. Fechtig et al., U.S. Patent No. 3,697,515, issued October 10, 1972, and L. Hatfield, U.S. Patent No. 3,868,368, issued February 25, 1975, all three of which are herein incorporated by reference.

An improved version of the above phosphorous pentachloride method employs a triphenylphosphite-chlorine kinetic reagent, as discussed in L. Hatfield et al., U.S. Patent No. 4,211,702, issued July 8, 1980, herein incorporated by reference. An excess (2 to 3 equivalents) of this reagent can be used to both cleave the 7-amido group and chlorinate the 3-hydroxy group of the 3-enol 1-carba-1-dethiacephem (for example, the compounds of Formula III above) analogous to the conditions set forth in L. Hatfield et al., U.S. Patent No. 4,226,986, issued October 7, 1980, herein incorporated by reference.

Yet another method for synthesizing the 7-amino compounds of Formula II is the method set forth in T. Hirata et al., U.K. Patent Application No. 2,041,923A and its equivalents (such as European Patent No. 14,475). This method entails, in general, addition of a phenylthiol to a 7-azido 3-hydro-1-carba-1-dethia-4-carboxylate compound. The resulting 3-thio-3,4-saturated compound is oxidized to the corresponding 3-sulfoxide compound. The 3-sulfoxide compound is chlorinated and the resultant 3-sulfoxide-3-chloro compound is treated with base (to eliminate the sulfoxide) to give the 3-chloro-3-cephem compound.

The 3-hydroxy starting materials (Formula III) of Scheme 1 are prepared as described in D.A. Evans et al., U.S. Patent No. 4,665,171, issued May 12, 1987, herein incorporated by reference. The 3-triflate analogs (Formula IIIa) are similarly described in U.S. Patent No. 4,673,737, also herein incorporated by reference.

In the following Preparations and Example, the terms nuclear magnetic resonance spectra, high performance liquid chromatography, field desorption mass spectroscopy, and specific rotation are abbreviated n.m.r., HPLC, f.d.m.s., and o.r., respectively.

In conjunction with the n.m.r. spectra, the following abbreviations are used: "s" is singlet, "d" is doublet, "dd" is doublet of doublets, "t" is triplet, "q" is quartet, and "m" is multiplet, respectively. "DMSO-$d_6$" is dimethyl sulfoxide where all protons have been replaced with deuterium.

The n.m.r. spectra were obtained on a Varian Associates EM-390 90 MHz instrument, on a Jeol FX-90Q 90 MHz instrument, on a Bruker Corporation 270 MHz or 500 MHz instrument, or on a General Electric QE-300 300 MHz instrument. The chemical shifts are expressed in δ values (parts per million downfield from tetramethylsilane).

## Preparation 1

m-(Methylsulfonylamino)benzaldehyde

m-(Amino)benzaldehyde polymer (1210 g, 10.0 mole), THF (12,000 ml), and water (495 ml) were combined and stirred overnight at room temperature. The resultant slurry was cooled to between about 5 to about 10°C and pyridine (1580 g, 20 moles) was added. Methylsulfonyl chloride (2290 g, 20 moles) was added in a dropwise fashion to the slurry while maintaining the temperature of reaction solution at approximately 10°C. The reaction was stirred in an ice bath overnight, during which time the ice in the bath melted and the temperature of the reaction climbed to between about 15° to 20°C. The THF was removed in vacuo and 1N hydrochloric acid (6 l) was added to the residue. The resultant slurry was stirred at room temperature for approximately 8 hours. The orange, granular material of the slurry was collected on a filter, was washed with water (approximately 6 liters) and was dried for 48 hours at 40°C to yield 1833 g of material. A portion of this material (940 g) was recrystallized from an ethyl acetate mixture containing carbon black and sodium carbonate. Two crops of crystals from this solution yielded 874 g of m-(methylsulfonylamino)benzaldehyde: n.m.r. (360 MHz, DMSO-$d_6$): δ 3.05 (s, 3), 7.5-7.9 (m, 4), 9.9 (s, 1), 10.1 (s, 1).

## Preparation 2

D,L-2-(amino)-2-(m-(methylsulfonylamino)phenyl)acetonitrile

Concentrated ammonium hydroxide (43 ml) was cooled to 10°C. Sodium cyanide (5.4 g, 110 mmol), ammonium chloride (5.5 g, 102 mmol), and m-(methylsulfonylamino)benzaldehyde (10.0 g, 50 mmol) were added and the resultant solution was stirred for 4 hours between about 10° to 15°C. Excess ammonia of the solution was removed in vacuo at 15°C. The pH of the residue was adjusted to 7.0 by the addition of concentrated hydrochloric acid. The resultant solution was extracted with ethyl acetate (6X) and the ethyl acetate extractions were combined, washed with brine (2X), dried over magnesium sulfate, filtered, and evaporated in vacuo to yield 10.6 g, 94% of a brown oil of D,L-2-(amino)-2-((m-methylsulfonylamino)phenyl)acetonitrile: n.m.r. (DMSO-$d_6$, 90 MHz): δ 3.0 (s, 3), 5.0 (s, 1), 7.2-7.6 (m, 4).

## Preparation 3

2-(R)-2-amino)-2-(m-(methylsulfonylamino)phenyl)acetonitrile, L-tartaric acid salt, acetic acid solvate

L-(+)-tartaric acid (8.25 g, 55 mmol) was dissolved in acetic acid (90%) and the mixture was heated to affect solution. The solution was added to D,L-2-(amino)-2-((m-methylsulfonylamino)phenyl)acetonitrile (10.6 g, 47 mmol) and the resultant solution was allowed to cool slowly. Ethyl acetate (25 ml) was slowly added and the resultant precipitate was stirred overnight at room temperature then was collected by filtration. The collected precipitate was washed with acetic acid and ethyl acetate then dried at 36°C in vacuo to yield 5 g, 49% of 2-(R)-2-(amino)-2-(m-(methylsulfonylamino)phenyl)acetonitrile, L-tartaric acid salt, acetic acid solvate: n.m.r.: ($D_2O$/DCl, 90 MHz): δ 2.1 (s, 1), 3.2 (s, 3), 4.8 (s, 1), 5.8 (s, 1), 7.3-7.7 (m, 4); o.r. $[\alpha]_D^{25} \triangleq +27.66°$ [1N hydrochloric acid, C 1].

## Preparation 4

2-(R)-2-amino-2-(m-(methylsulfonylamino)phenyl)acetic acid, hydrochloride salt

2-(R)-2-(amino)-2-(m-(methylsulfonylamino)phenyl)acetonitrile, L-tartaric acid salt, acetic acid solvate (18 g, 48 mmol) was heated to reflux temperature in 6N hydrochloric acid (200 ml) for approximately 6 hours. After removal of excess hydrochloric acid under reduced pressure, carbon black was added and the suspension was filtered through a filter cell. The pH of the filtrate was adjusted to 5.0 by the addition of 5N sodium hydroxide solution. The filtrate was used in situ in Preparation 5.

## Preparation 5

2-(R)-(N-(t-butoxycarbonylamino)-2-(m-(methylsulfonylaminophenyl)acetic acid

The pH of the solution from Preparation 4 above was adjusted to 9 by the addition of 5N sodium hydroxide solution. THF (300 ml) then di(t-butyl dicarbonate) (15.7 g, 72 mmol) was added and the pH of the solution was readjusted to 9.0 by the addition of 5N sodium hydroxide solution. The solution was stirred at room temperature for 48 hours then the THF was removed in vacuo. The resultant concentrate was washed with diethyl ether (2X), and the pH of the water layer was adjusted to 2 by the addition of 6N hydrochloric acid. The acidified aqueous layers were extracted with ethyl acetate (4X). The ethyl acetate layers were combined, dried over magnesium sulfate, filtered, and evaporated in vacuo to give 11 g of foam. The foam was

chromatographed by preparatory-scale HPLC on a silica column eluted with a gradient of toluene versus 50% ethyl acetate/toluene to give 6.85 g, 41% of an 84:16 R:S mixture of 2-(R)-(N-(t-butoxycarbonylamino))-2-(m-(methylsulfonylamino)phenyl)acetic acid: n.m.r. (300 MHz, $CDCl_3$): 1.3 (s, 9), 5.1 (d, 1), 5.3 (d, 1), 7.1-7.5 (m, 4), 7.9 (d, 1); o.r.: $[\alpha]_D^{25°} =$ -74.4° [methanol, C 1].

Preparation 6

p-Nitrobenzyl 7β-[2′-(R)-2′-(m-(methylsulfonamido)phenyl)-2′-(N-(t-butoxycarbonylamino))acetamido]-3-chloro-3-(1-carba-1-dethiacephem)-4-carboxylate

p-Nitrobenzyl 7β-amino-3-chloro-3-(1-carba-1-dethiacephem)-4-carboxylate hydrochloride salt (194 mg, 0.5 mmol) was suspended in methylene chloride (3.5 ml) then N-methylmorpholine (55.5 μl) was added. 2-(R)-2-(m-methylsulfonamido)phenyl)-2-[N-(t-butoxycarbonylamino)]acetic acid (172 mg, 0.5 mmol) was added and the solution was cooled to 0° C. Pyridine (170 μl, 2.1 mmol) then phosphoryl chloride (79.1 μl, 0.85 mmol) was added and the solution was stirred for 2 hours at approximately 0° to approximately 5° C. 1N Hydrochloric acid (10 ml) was added and the methylene chloride was removed under reduced pressure. Ethyl acetate (50 ml) was added and the solution was washed with 1N hydrochloric acid (2X), brine, dried over magnesium sulfate, filtered, and evaporated in vacuo to yield 245 mg of a foam. The foam was chromatographed by HPLC on a silica gel column, eluted with a mixture of 48%/48%/4% ethyl acetate/hexane/isopropanol to yield 165 mg, 49% of p-nitrobenzyl 7β-[2′-(R)-2′-(m-(methylsulfonamido)phenyl)-2′-(N-(t-butoxycarbonylamino))acetamido]-3-chloro-3-(1-carba-1-dethiacephem)-4-carboxylate: n.m.r. (300 MHz, $CDCl_3$): δ 1.4 (s, 9), 1.6-1.9 (m, 2), 2.5-2.8 (m, 2), 3.0 (s, 3), 3.8-4.0 (m, 1), 5.2 (d, 1), 5.4 (q, 2), 5.9 (q, 1), 7.1-7.4 (m, 4), 7.6 (d, 2), 8.2 (d, 2).

Preparation 7

7β-[2′-(R)-2′-(m-(methylsulfonamido)phenyl)-2′-(N-(t-butoxycarbonylamino))acetamido]-3-chloro-3-(1-carba-1-dethiacephem)-4-carboxylic acid

Five percent palladium on carbon (150 mg) was wetted with ethanol, then p-nitrobenzyl 7β-[2′-(R)-2′-(m-(methylsulfonamido)phenyl)-2-(N-(t-butoxycarbonylamino))acetamido]-3-chloro-3-(1-carba-1-dethiacephem)-4-carboxylate (300 mg, 0.44 mmol) (dissolved in a minimum amount of ethyl acetate) was added. The resultant suspension was hydrogenated for 20 minutes at a hydrogen pressure of 40 psi. The suspension was filtered, then washed with 1N hydrochloric acid (3X), brine (2X), dried over magnesium sulfate, filtered, and evaporated in vacuo to a solid. The solid was stirred in diethyl ether for 48 hours then collected by filtration and dried in vacuo. The solid was crystallized from diethyl ether, triturated with diethyl ether, filtered, then dried in vacuo to yield 50 mg, 62% of 7β-[2-(R)-2-(m-(methylsulfonamido)phenyl)-2-[N-(t-butoxycarbonylamino)acetamido]-3-chloro-3-[1-carba-1-dethiacephem]-4-carboxylic acid: f.d.m.s.: $M^+ + 1 = 543$.

Preparation 8

7β-[2′-(R)-2′-(m-(methylsulfonamido)phenyl)-2′-aminoacetamido]-3-chloro-3-(1-carba-1-dethiacephem)-4-carboxylic acid trifluoroacetate salt

Trifluoroacetic acid (approximately 10 ml) was cooled to approximately 0° C. 7β-[2′-(R)-2′-(m-(methylsulfonamido)phenyl)-2′-[N-(t-butoxycarbonylamino)acetamido]-3-chloro-3-(1-carba-1-dethiacephem)-4-carboxylic acid (397 mg, 0.73 mmol) was added and the resultant solution was stirred at approximately 0° C for 20 minutes then warmed to room temperature. The solvent was evaporated in vacuo to give an oil. The oil was combined with diethyl ether and the resultant solid was triturated for 90 minutes with diethyl ether. The mixture was filtered and the solid dried in vacuo to yield 392 mg, 96% of 7β-[2′-(R)-2′-(m-(methylsulfonamido)phenyl)-2′-aminoacetamido]-3-chloro-3-(1-carba-1-dethiacephem)-4-carboxylic acid trifluoroacetate salt: n.m.r. (300 MHz, DMSO-$d_6$): δ 1.4 (m, 2), 2.3 - 2.7 (m, 2), 3.0 (s, 3), 3.8 (m, 1), 4.9 (s, 1), 5.4 (s, 1), 7.1-7.5 (m, 4), 3.3 (s, 2), 9.9 (broad, 1).

Preparation 9

7β-[2′-(R)-2′-(m-(methylsulfonamido)phenyl)-2′-aminoacetamido]-3-chloro-3-(1-carba-1-dethiacephem)-4-carboxylic acid, zwitterionic salt (LY228238)

7β-[2′-(R)-2′-(m-(methylsulfonamido)phenyl)-2′-aminoacetamido]-3-chloro-3-(1-carba-1-dethiacephem)-4-carboxylic acid trifluoroacetate salt was chromatographed by preparatory-scale HPLC on a 20 μ $C_{18}$ reverse-phase silica column eluted with a gradient of 5% to 15% acetonitrile/1% acetic acid/water to yield 73.6 mg of 7β-[2′-(R)-2′-(m-(methylsulfonamido)phenyl)-2′-aminoacetamido]-3-chloro-3-(1-carba-1-dethiacephem)-4-carboxylic acid, zwitterionic salt: n.m.r. (270 MHz, DMSO-$d_6$) δ 1.48 (m, 2), 2.33 (m, 2), 2.99 (s, 3), 3.73 (m, 1, J = 4.7, 5.0, 10.0 Hz), 4.64 (s, 1), 5.26 (d, 1, J = 4.7 Hz), 7.1-7.4 (m, 4).

## Example 1

7β-(2′-(R)-2′-(m-(methylsulfonamido)phenyl)-2′-aminoacetamido]-3-chloro-3-(1-carba-1-dethiacephem)-4-carboxylic acid 2.5 hydrate (LY228238 2.5 hydrate)

A 208 mg sample of LY228238 was dissolved in 1% acetic acid in water and the resulting solution freeze-dried. The solid was dissolved in 2 ml of water at 38°C. The solution was allowed to sit at room temperature for 16 hours. The resulting crystals were recovered by vacuum filtration, rinsed with room temperature water, and air dried to provide the desired title product. The product provided an X-ray powder diffraction pattern as reported in the Table above.

Analysis for ($C_{17}H_{19}ClN_4O_6S$) · 2.5 $H_2O$:

| | | | |
|---|---|---|---|
| Calc.: | C, 41.85; | H, 4.96; | N, 11.48; |
| Found: | C, 41.56; | H, 4.86; | N, 11.31. |

Karl Fischer analysis of other lots of product prepared in like manner confirmed the presence of 2.5 moles of water per mole of LY228238.The following examples illustrate pharmaceutical formulations containing the compound of this invention as the active ingredient.

## Example 2

Hard gelatin capsules are prepared using the following ingredients:

| | Quantity (mg/capsule) |
|---|---|
| LY228238 2.5 hydrate | 200 |
| Starch flowable powder | 186 |
| Starch flowable powder with silicone 5% | 50 |
| Magnesium stearate | 2.5 |

The above ingredients are mixed and filled into hard gelatin capsules in 438.5 mg quantities.

## Example 3

A tablet formula is prepared using the ingredients below:

| | Quantity (mg/tablet) |
|---|---|
| LY228238 2.5 hydrate | 200 |
| Cellulose, microcrystalline | 200 |
| Silicon dioxide, fumed | 10 |
| Stearic acid | 5 |

The components are blended and compressed to form tablets each weighing 415 mg.

## Example 4

Suppositories each containing 200 mg of active ingredient are made as follows:

| | |
|---|---|
| LY228238 2.5 hydrate | 200 mg |
| Saturated fatty acid glycerides to | 2000 mg |

The active ingredient is passed through a No. 60 mesh U.S. sieve and suspended in the saturated fatty acid glycerides previously melted using the minimum heat necessary. The mixture is then poured into a suppository mold of nominal 2 g capacity and allowed to cool.

## Example 5

### Ready To-Use Aqueous Suspension

| | |
|---|---|
| LY228238 2.5 hydrate | 210 mg |
| Cellulose with sodium carboxymethylcellulose | 95 mg |
| sucrose | 1.85 g |
| parabens | 3 mg |
| emulsion silicone | 2.5 mg |
| pluronic | 5 mg |
| flavor | 2 mg |
| color | 0.5 mg |
| purified water | q.s. to 5 ml |

The ingredients are sieved. The parabens are dissolved in hot purified water and, after cooling, the other ingredients are added. Sufficient purified water is added to produce the required volume. The suspension can then be passed through a colloid mill or homogenizer to produce a more elegant dispersion.

## Example 6

### Suspension for Reconstitution

| | |
|---|---|
| LY228238 2.5 hydrate | 210 mg |
| Sucrose | 3 g |
| Xanthan gum | 5 mg |
| starch modified | 10 mg |
| emulsion silicone | 5 mg |
| sodium lauryl sulfate | 0.5 mg |
| methylcellulose | 2 mg |
| flavor | 0.5 mg |
| dye | |

The ingredients are sieved and mixed in a suitable blender, such as a twin-shell, twin core Nauta®-type, or ribbon blender. To constitute, a sufficient volume of purified water is added to produce the required volume.

**Claims**

1. A crystalline 2.5 hydrate form of the compound of the Formula (I)

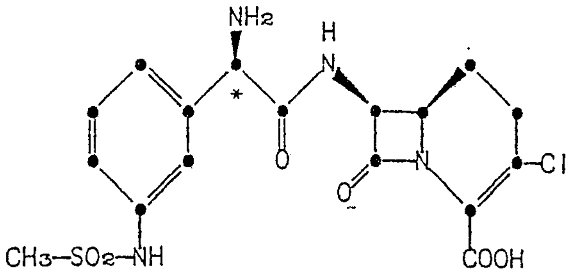

(I)

2. A pharmaceutical formulation comprising as an active ingredient a crystalline 2.5 hydrate of the compound of the Formula (I) as claimed in Claim 1, associated with one or more pharmaceutically-acceptable carriers, vehicles or excipients therefor.

3. A crystalline 2.5 hydrate of the compound of the Formula (I) as claimed in Claim 1 for use in treating bacterial infections in warm-blooded animals.

4. A process for preparing the crystalline 2.5 hydrate of the compound of Formula (I) as claimed in Claim 1 which comprises freeze-drying an aqueous solution containing a compound of Formula (I) and crystallizing the resulting solid from water.

**Claims for the following Contracting States : GR, ES**

1. A process for preparing the crystalline 2.5 hydrate form of the compound of the Formula (I)

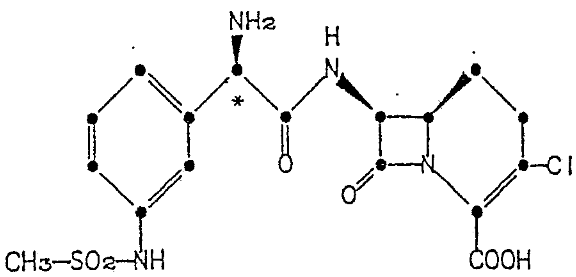

(I)

which comprises freeze-drying an aqueous solution containing a compound of Formula (I) and crystallizing the resulting solid from water.

2. A process for preparing a pharmaceutical formulation comprising admixing a crystalline 2.5 hydrate of the compound of the Formula (I) as defined in Claim 1, with one or more pharmaceutically-acceptable carriers, vehicles or excipients therefor.

European Patent Office

**PARTIAL EUROPEAN SEARCH REPORT**
which under Rule 45 of the European Patent Convention shall be considered, for the purposes of subsequent proceedings, as the European search report

Application number
EP 89 30 1011

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,A | EP-A-0 014 476 (KYOWA HAKKO KOGYO CO.)<br>* Claims *<br>-- | 1,2 | C 07 D 471/04<br>A 61 K 31/435//<br>(C 07 D 471/04,<br>221/00,<br>205/00) |
| D,P,A | EP-A-0 266 896 (ELI LILLY)<br>* Claims *<br>------- | 1,2 | |
| | | | TECHNICAL FIELDS SEARCHED (Int. Cl.4)<br>C 07 D 471/00<br>501/00<br>A 61 K 31/00 |

## INCOMPLETE SEARCH

The Search Division considers that the present European patent application does not comply with the provisions of the European Patent Convention to such an extent that it is not possible to carry out a meaningful search into the state of the art on the basis of some of the claims.
Claims searched completely: 1,2,4
Claims searched incompletely:
Claims not searched: 3
Reason for the limitation of the search:

Method for treatment of the human or animal body by surgery or therapy (See art. 52(4) of the European Patent Convention)

| Place of search | Date of completion of the search | Examiner |
|---|---|---|
| The Hague | 25-04-1989 | CHOULY |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document